(19) Europäisches Patentamt
European Patent Office
Office européen des brevets

(11) Veröffentlichungsnummer: **0 003 795**
**B1**

# (12) EUROPÄISCHE PATENTSCHRIFT

(45) Veröffentlichungstag der Patentschrift: **14.10.81**

(51) Int. Cl.³: **C 07 C 102/00,** C 07 C 103/34

(21) Anmeldenummer: **79100420.3**

(22) Anmeldetag: **13.02.79**

(54) **Verfahren zur Herstellung von N-substituierten Carbonsäureamiden.**

(30) Priorität: **23.02.78 DE 2807659**

(43) Veröffentlichungstag der Anmeldung: **05.09.79 Patentblatt 79/18**

(45) Bekanntmachung des Hinweises auf die Patenterteilung: **14.10.81 Patentblatt 81/41**

(84) Benannte Vertragsstaaten: **BE CH DE FR GB NL**

(56) Entgegenhaltungen:
**»ORGANIC REACTIONS« Band 17, 1969 JOHN WILEY & SONS, INC, New York**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubensstrasse 25, 6700 Ludwigshafen (DE)**
Erfinder: **Kempe, Uwe, Dr., Carl-Bosch-Strasse 44, 6703 Limburgerhof (DE)**

**EP 0 003 795 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von N-substituierten Carbonsäureamiden

Die Erfindung betrifft ein Verfahren zur Herstellung von N-substituierten Carbonsäureamiden durch Umsetzung von Cyanverbindungen mit Olefinen und Wasser in Gegenwart von sulfonsauren Kationenaustauschern.

Es ist aus Houben-Weyl, Methoden der Organischen Chemie, Band 11/1, Seiten 994 bis 996, bekannt, daß man Blausäure oder Nitrile mit Olefinen in Gegenwart von Schwefelsäure zu N-Formylaminen bzw. N-substituierten Carbonsäureamiden umsetzt. Durch Hydrolyse gelangt man dann zu den entsprechenden Aminen; auf diese elegante Weise sind zahlreiche Amine, insbesondere tert.-Alkylamine, die auf andere Weise nicht oder nur schwer zugänglich sind, herstellbar. Die Umsetzung wird in den Beispielen mit äquimolaren oder überschüssigen Mengen an Schwefelsäure durchgeführt.

Bevorzugt verwendet man bei der Reaktion Schwefelsäure, wie Organic Reactions, Band 17 (1969), Seiten 213 bis 325, angibt, jedoch wurden auch schon als Katalysatoren Essigsäure, Salzsäure, Benzolsulfonsäure, Toluolsulfonsäure, Propionsäure, Bortrifluoridhydrat, Polyphosphorsäure, Fluorborsäure, Ferrocyanwasserstoffsäure, Phosphorsäure und Ameisensäure angewendet (loc. cit., Seiten 257 bis 324).

Ein wesentlicher Nachteil dieser bis jetzt bekannten Verfahren ist neben der Korrosivität der Säuren die notwendige große Säuremenge, in der Regel 1 bis 4 Mol Säure je Mol Olefin, wie auch die DE-AS 11 96 185 und DE-AS 21 44 230 zeigen. Die Säure zwingt zu einer aufwendigen und umweltbelastenden Aufarbeitung des Reaktionsgemisches. Zwecks Isolierung der Amide muß mit Wasser verdünnt und neutralisiert werden, was den Verlust der Säure und eine Abwasserbelastung durch große Salzmengen bedeutet. Anschließend muß das Gemisch filtriert bzw. mit einem geeigneten Lösungsmittel, z. B. Äther (DE-AS 11 96 185), extrahiert werden. Bei einer weiteren Aufarbeitungsweise wird nach dem Verdünnen des Reaktionsgemisches mit geeigneten Phosphorsäureestern oder Harnstoffderivaten extrahiert, wobei der Extrakt zur Entfernung von Säurespuren gegebenenfalls mit Wasser gewaschen werden muß (DAS 21 44 230). Die Rückgewinnung des Extraktionsmittels bzw. die Isolierung des Amids erfolgt insbesondere durch Destillation im Vakuum.

Alle diese Verfahren befriedigen mit Bezug auf Raum-Zeit-Ausbeute und einfachen und wirtschaftlichen Betrieb, insbesondere auch in Hinsicht auf die Aufarbeitung des Reaktionsgemisches nicht.

Es wurde nun gefunden, daß man N-substituierte Carbonsäureamide der Formel

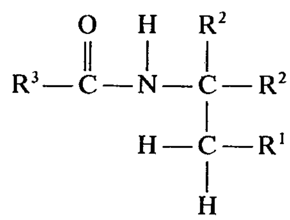

(I)

worin die einzelnen Reste $R^1$ und $R^2$ gleich oder verschieden sein können und jeweils einen aliphatischen Rest bedeuten, zwei der Reste $R^1$ und $R^2$ auch jeweils für ein Wasserstoffatom stehen können, $R^3$ ein Wasserstoffatom, einen aliphatischen, araliphatischen oder aromatischen Rest bezeichnet, durch Umsetzung von Olefinen mit Cyanverbindungen vorteilhaft erhält, wenn man Olefine der Formel

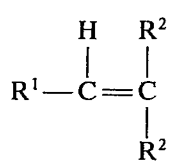

(II)

worin $R^1$ und $R^2$ die vorgenannte Bedeutung besitzen, mit Cyanverbindungen der Formel

$$R^3CN \qquad \text{(III)}$$

worin $R^3$ die vorgenannte Bedeutung besitzt, und Wasser in Gegenwart von organischen, sulfonsauren Kationenaustauschern in einer Menge von 10 bis 80 Gewichtsprozent Austauscher, bezogen auf Ausgangsstoff II, umsetzt.

Die Umsetzung kann für den Fall der Verwendung von Acetonitril und Isobutylen durch die folgenden Formeln wiedergegeben werden:

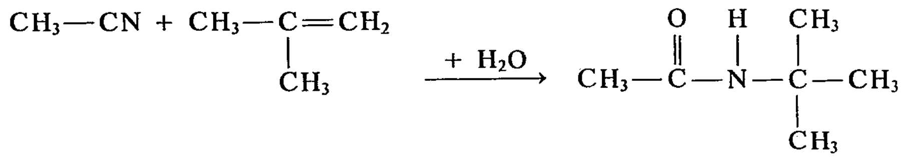

Im Vergleich zu den bekannten Verfahren liefert das Verfahren nach der Erfindung überraschend auf einfacherem, wirtschaftlicherem und umweltfreundlicherem Wege eine große Zahl von N-substituierten Carbonsäureamiden in guter Raum-Zeit-Ausbeute, gerade auch im großtechnischen Maßstab und im kontinuierlichen Betrieb, und teilweise besserer Ausbeute und Reinheit. Die Katalysatoren sind wesentlich leichter zu isolieren und wiederzuverwerten. Alle diese Vorteile des erfindungsgemäßen Verfahrens sind überraschend insbesondere auch im Hinblick darauf, daß nur kleine Katalysatormengen verwendet werden.

Der Ausgangsstoff II kann mit der Cyanverbindung III in stöchiometrischer Menge oder im Überschuß, vorzugsweise in einem Verhältnis von 0,1 bis 10, insbesondere 0,5 bis 5 Mol Ausgangsstoff III je Mol Ausgangsstoff II, umgesetzt werden. Wasser gelangt in stöchiometrischer Menge oder im Überschuß, zweckmäßig in einem Verhältnis von 0,2 bis 5, vorzugsweise von 0,5 bis 2 Mol Wasser je Mol Ausgangsstoff II, zur Anwendung.

Bevorzugte Ausgangsstoffe II, III und dementsprechend bevorzugte Endstoffe I sind solche, in deren Formeln die einzelnen Reste $R^1$ und $R^2$ gleich oder verschieden sein können und jeweils einen Alkylrest mit 1 bis 9, insbesondere 1 bis 4 Kohlenstoffatomen bedeuten, zwei der Reste $R^1$ und $R^2$ auch jeweils für ein Wasserstoffatom stehen können, $R^3$ ein Wasserstoffatom, einen Alkylrest mit 1 bis 9, insbesondere 1 bis 4 Kohlenstoffatomen, einen Alkenylrest mit 2 bis 9, insbesondere 2 bis 4 Kohlenstoffatomen, einen Alkylphenylrest oder Phenylalkylrest mit 7 bis 12 Kohlenstoffatomen oder einen Phenylrest bezeichnet. Die vorgenannten Reste können noch durch unter den Reaktionsbedingungen inerte Gruppen, z. B. Alkylgruppen mit 1 bis 3 Kohlenstoffatomen, substituiert sein. Bevorzugt verwendet man verzweigte Alkene.

Anstelle der Olefine II können auch Stoffe, die unter den Reaktionsbedingungen ein Olefin durch Wasserabspaltung bilden, verwendet werden, z. B. tertiäre oder sekundäre Alkanole, zweckmäßig Alkanole der Formel

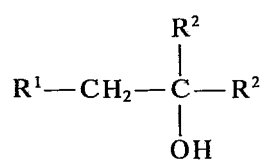
(IIa)

worin $R^1$ und $R^2$ die vorgenannte Bedeutung besitzen. Da Wasser gebildet wird, kann man in solchen Fällen der erfindungsgemäßen Reaktion vorteilhaft auf einen Wasserzusatz ganz oder teilweise verzichten.

Ebenfalls kommen auch Stoffe, die unter den Reaktionsbedingungen ein Olefin bilden, aber nicht Wasser abspalten, anstelle der Ausgangsstoffe II in Frage, z. B. tertiäre und sekundäre Alkylester und Alkyläther, zweckmäßig solche der Formel

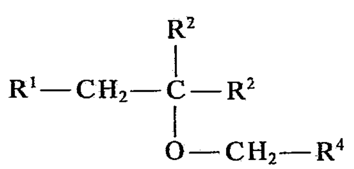
(IIb)

und

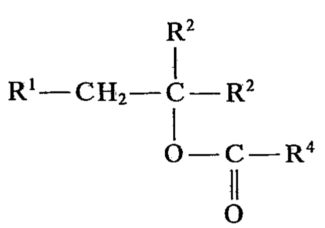
(IIc)

worin $R^1$ und $R^2$ die vorgenannte Bedeutung besitzen und die Reste $R^4$ jeweils ein Wasserstoffatom oder einen aliphatischen Rest, vorzugsweise einen Alkylrest mit 1 bis 9, insbesondere 1 bis 4

Kohlenstoffatomen bezeichnen. In diesen Fällen muß bei der Reaktion Wasser zugesetzt werden, da sich kein Wasser abspaltet.

Es können z. B. folgende Olefine als Ausgangsstoffe II verwendet werden: n-Penten-(1), n-Hexen-(1), n-Hepten-(1), n-Octen-(1), n-Nonen-(1), n-Decen-(1), n-Undecen-(1), n-Dodecen-(1), Propen, n-Buten-(1); vorgenannte Alkene, die in 2-Stellung oder 3-Stellung oder 4-Stellung durch die Methyl-, Äthyl-, n-Propyl-, Isopropyl, n-Butyl-, Isobutyl-, sek.-Butyl-, tert.-Butyl-gruppe substituiert sind; 2,3-Dimethyl-n-buten, 3,3-Dimethyl-n-buten, 2,5-Dimethylhepten, 3,3-Dimethylhepten, 2,3,4-Trimethylhepten, 2,4-Dimethylhepten, 2,3-Dimethylhepten, 4,4-Dimethylhepten, 2,3-Diäthylhexen, 4,4-Dimethylhexen, 2,3-Dimethylhexen, 2,4-Dimethylhexen, 2,5-Dimethylhexen, 3,3-Dimethylhexen, 3,4-Dimethylhexen, 2-Methyl-3-äthylpenten, 3-Methyl-3-äthylpenten, 2,3,3-Trimethylhepten, 2,4,4-Trimethylpenten, 2,3,3-Trimethylpenten, 2,3,4-Trimethylpenten, 2,3,3,4-Tetramethylhexen; analoge Alkene, deren Doppelbindung in 2-Stellung oder in 3-Stellung im Molekül liegen; verzweigte Alkene, wie sie in Gestalt von Gemischen bei der Dimerisierung von Isobutylen oder n-Buten (Octene) oder Trimerisierung von Isobutylen oder n-Buten (Dodecene) oder Propylen (Nonene) bzw. Tetramerisierung von Propylen (Dodecene) anfallen. Bevorzugt sind: Isobutylen, n-Buten, Propen, 2,3-Dimethyl-buten-1, 2-Methyl-buten-1, 2-Methyl-buten-(2), 2-Methyl-penten-2, 3-Methyl-hexen-3, 2-Methyl-hepten-1, 2,3-Dimethyl-penten-1, 2,3-Dimethyl-hexen, 2,4,4-Trimethylpenten-1.

Entsprechend den vorgenannten Olefinen II kommen die analogen tertiären und sekundären Alkanole, Alkylester und Alkyläther in Betracht, bevorzugt tert.-Butanol, Isopropanol, sek.-Butanol, tert.-Butylacetat und Methyl-tert.-butyläther.

Beispielsweise kommen folgende Cyanverbindungen III in Betracht: Blausäure, Isopropionitril, Butyronitril, Isobutyronitril, sek.-Butyronitril, tert.-Butyronitril, Acetonitril, Propionitrile, Acrylnitril, Benzonitril, Phenylacetonitril.

Die Umsetzung wird im allgemeinen bei einer Temperatur von 50 bis 150° C, vorzugsweise von 70 bis 140° C, insbesondere von 75 bis 125° C, mit Unterdruck, Überdruck oder drucklos, diskontinuierlich oder kontinuierlich durchgeführt. Die Verweilzeit im kontinuierlichen Betrieb beträgt vorzugsweise von 0,5 bis 20, insbesondere von 1 bis 10 Stunden, der Durchsatz im kontinuierlichen Betrieb von 1 bis 60, insbesondere von 2 bis 30 Kilogramm Ausgangsstoff II je Kilogramm Katalysator und Stunde. Der Austauscher gelangt in einer Menge von 10 bis 80, vorzugsweise 40 bis 70 Gewichtsprozent Austauscher, bezogen auf Ausgangsstoff II, zur Anwendung.

Als Katalysatoren werden organische, sulfonsaure Kationenaustauscher, vorteilhaft Harze aus sulfoniertem Polystyrol oder sulfonierten, vernetzten Styrolpolymeren; bifunktionelle, sulfonsaure Kondensationsharze; Phenolsulfonsäureharze; sulfonsaure Phenolformaldehydharze; verwendet. Vorzugsweise kommen sulfonierte Polystyrol-divinylbenzolaustauscher in Frage. Die Austauscher liegen in der Säureform und nicht als Salz vor. Der Katalysator kann grobkörnig oder zweckmäßig feinkörnig sein und hat zweckmäßig eine Korngröße von 0,005 bis 2, vorteilhaft 0,02 bis 1 Millimeter. Er hat zweckmäßig gelartige Struktur. Geeignet sind z. B. Austauscherharze, wie sie unter der Bezeichnung ®LEWASORB A-10, im Handel erhältlich sind. Ebenfalls kann man handelsübliche Harze ®, z. B. ®Amberlit IR-120, ®Dowex 50, ®Lewatit S-100, ®Nalcite HCR, ®Permutit RS, ®Wofatit KPS-200, ®Amberlyst-15, ®Lewatit SC-108, ®Lewatit SPC-108, verwenden. Man entwässert sie zweckmäßig vor der Verwendung in üblicher Weise, z. B. durch Erhitzen im Vakuum. Die Entwässerung kann jedoch auch durch Verdrängen mit hydrophilen organischen Flüssigkeiten und anschließendem Erhitzen auf 100° C bei vermindertem Druck oder durch azeotrope Destillation mit einer organischen Flüssigkeit erfolgen.

Der Katalysator befindet sich während der Umsetzung vorteilhaft in Suspension, in der Regel im sich bildenden Reaktionsgemisch. Zweckmäßig legt man einen Anteil des Ausgangsgemisches an Cyanverbindung III und Olefin II vor und suspendiert den Katalysator in der Flüssigkeit unter guter Durchmischung. Zweckmäßig verwendet man keine zusätzlichen Lösungsmittel; gegebenenfalls kommen aber auch, z. B. zur Erniedrigung der Viskosität des Reaktionsgemisches, unter den Reaktionsbedingungen inerte Lösungsmittel in Betracht. Als Lösungsmittel kommen z. B. in Frage: aliphatische oder cycloaliphatische Kohlenwasserstoffe, z. B. Heptan, Nonan, Benzinfraktionen innerhalb eines Siedepunktintervalls von 70 bis 190° C, Cyclohexan, Methylcyclohexan, Dekalin, Petroläther, Hexan, Ligroin, 2,2,4-Trimethylpentan, 2,2,3-Trimethylpentan, 2,33,3-Trimethylpentan, Octan; Halogenkohlenwasserstoffe, insbesondere Chlorkohlenwasserstoffe, z. B. Tetrachloräthylen, 1,1,2,2-Tetrachloräthan oder 1,1,1,2-Tetrachloräthan, Dichlorpropan, Methylenchlorid, Dichlorbutan, Chloroform, Tetrachlorkohlenstoff, Tetrachloräthan, 1,1,1-Trichloräthan oder 1,1,2-Trichloräthan, Trichloräthylen, Pentachloräthan, cis-Dichloräthylen, 1,2-Dichloräthan, 1,1-Dichloräthan; Diäthyläther, Tetrahydrofuran, Dioxan; und entsprechende Gemische. Zweckmäßig verwendet man das Lösungsmittel in einer Menge von 5 bis 500 Gewichtsprozent, vorzugsweise von 20 bis 100 Gewichtsprozent, bezogen auf Ausgangsstoff II. Man kann im Falle von Gemischen an Ausgangsstoffen II, z. B. aus der Erdölcrackung, die im Gemisch enthaltenen gesättigten Kohlenwasserstoffe als Lösungsmittel für die Suspension verwenden. Man wählt die Menge an vorgelegtem Ausgangsgemisch und/oder organischem Lösungsmittel vorteilhaft so, daß der Katalysator im sich bildenden Reaktionsgemisch in einer Menge von 1 bis 30, vorzugsweise von 10 bis 20 Gewichtsprozent, bezogen auf das Gewicht des gesamten flüssigen Gemisches im Reaktionsraum,

suspendiert ist. Zweckmäßig wird das Reaktionsgemisch während der gesamten Umsetzung durchmischt, vorzugsweise mit einer Rührgeschwindigkeit von mindestens 100, zweckmäßig von 200 bis 2000, insbesondere von 300 bis 1000 Umdrehungen pro Minute. Bei Durchmischungseinrichtungen ohne Rührwerk, z. B. auch bei Durchmischung mittels Inertgas wie Stickstoff, werden solche bevorzugt, die vorgenannter Rührgeschwindigkeit entsprechende Scherenergie einbringen. Auf diese Weise wird eine feindisperse Suspension erzielt. Unter Verwendung vorgenannter Durchmischungsbedingungen können in einem breiten Maße bekannte Rührvorrichtungen verwendet werden: Injektoren, Kugeldüsen, Dralldüsen, Turbinenrührer, Mischdüsen, Lechler-Mischdüse, Blattrührer, Ankerrührer, Balkenrührer, Propellerrührer, Cramer-Rührer, Vibromischer, Fingerrührer, Kreuzbalkenrührer, Kreiselrührer, Gitterrührer, Flachrührer, Spiralturbinen, Schaufelblattrührer, Planetenrührwerke, Zentrifugalrührkreisel, rotierende Zerstäuber, Strahldüsen, Dreikantrührer, Hohlrührer, Rohrrührer, Impeller-Rührer. Ebenfalls kommen Apparaturen und Anlagen, wie Rührkessel, Rührkesselkaskaden, Strömungsrohre, Mammutrührwerke, Homogenisiermaschinen, Kreiselmischer, Turbomischer, Emulgierzentrifugen, Ultraschallflüssigkeitspfeife, Durchlaufmischer, Drehtrommeln, Kammerreaktoren, Kreislaufreaktoren, Schlaufenreaktoren, Zellarreaktoren, Schneckenreaktoren, Blasensäulen, in Frage; bevorzugt sind schon aus wirtschaftlichen Gründen Rührkessel.

Die Reaktion kann wie folgt durchgeführt werden: Ein Gemisch von Ausgangsstoff II und III, Wasser, Katalysator, gegebenenfalls zusammen mit Lösungsmittel, wird bei der Reaktionstemperatur, im diskontinuierlichen Betrieb während 1 bis 6 Stunden, umgesetzt, wobei der Katalysator im Ausgangsgemisch bzw. Reaktionsgemisch in Suspension gehalten wird. Dann wird aus dem Reaktionsgemisch der Endstoff in üblicher Weise, z. B. durch Filtration und Destillation, abgetrennt. Die Abtrennung des Ionenaustauschers in diskontinuierlicher Fahrweise kann z. B. durch Filtrieren, Abschleudern oder Sedimentieren erfolgen, der Katalysator kann ohne weiteres erneut verwendet werden.

Bei einer vorteilhaften Form der kontinuierlichen Durchführung leitet man das Gemisch der Ausgangsstoffe und des Katalysators und gegebenenfalls des Lösungsmittels kontinuierlich in einen Reaktor, in dem sich eine Suspension des Katalysators im Reaktionsgemisch befindet, und entnimmt gleichzeitig eine adäquate Menge Suspension kontinuierlich über Filter. Die Filtrierung erfolgt zweckmäßig vor Austritt der Suspension aus dem Reaktor. Als Filter kommen säurefeste Filtertücher, Drahtnetzfilter, Sintermetallfilter, sofern die Maschenweiten bzw. Porendurchmesser kleiner sind als die Katalysatorpartikel, in Betracht. Ebenso können zweckmäßig Dekantierapparate zur Katalysatorabtrennung verwendet werden. Der abgetrennte Katalysator wird in der Regel kontinuierlich der Reaktion wieder zugeführt.

Die nach dem Verfahren der Erfindung herstellbaren Endstoffe I sind wertvolle Ausgangsstoffe für die Herstellung von Farbstoffen, Schädlingsbekämpfungsmitteln, Emulgatoren, Dispergiermittel, Stabilisatoren, Textilhilfsmitteln wie Druckereihilfsmitteln. Bezüglich der Verwendung wird auf die vorgenannten Veröffentlichungen, Ullmanns Encyklopädie der technischen Chemie, Band 5, Seite 108 und Ergänzungsband, Seite 136, verwiesen.

Die in den folgenden Beispielen aufgeführten Teile bedeuten Gewichtsteile.

## Beispiel 1

In einem Rührautoklaven wird eine Suspension von 123 Teilen Acetonitril, 74 Teilen tert.-Butanol und 25 Teilen Austauscherharz mit einer Rührgeschwindigkeit von 500 Umdrehungen pro Minute hergestellt und auf 120°C erhitzt. Das Austauscherharz ist ein sulfoniertes, aus Styrol und Divinylbenzol copolymerisiertes Harz, das vor der Verwendung während 20 Stunden bei 100°C im Vakuum entwässert wurde; es hat Gelstruktur und besitzt eine Korngröße von 20 bis 200 Mikrometern. Nach 3 Stunden Rühren bei 120°C wird der Katalysator abgetrennt und das Filtrat destilliert. Als Rückstand erhält man 175 Teile N-tert.-Butylacetamid (89% der Theorie, bezogen auf eingesetztes tert.-Butanol) vom Fp 97 bis 98°C.

## Beispiel 2

In einem Rührautoklaven wird eine Suspension von 123 Teilen Acetonitril, 56 Teilen Isobuten, 18 Teilen Wasser und 30 Teilen Austauscherharz mit einer Rührgeschwindigkeit von 500 Umdrehungen pro Minute hergestellt und auf 120°C erhitzt. Das Austauscherharz ist ein sulfoniertes, aus Styrol und Divinylbenzol copolymerisiertes Harz, das vor der Verwendung während 20 Stunden bei 100°C im Vakuum entwässert wurde; es hat Gelstruktur und besitzt eine Korngröße von 20 bis 200 Mikrometern. Nach 4 Stunden Rühren bei 120°C wird der Katalysator abgetrennt und das Filtrat destilliert. Als Rückstand erhält man 187 Teile N-tert.-Butylacetamid (95% der Theorie, bezogen auf Isobuten) vom Fp 97 bis 98°C.

## Beispiel 3

In einem Rührautoklaven wird eine Suspension von 148 Teilen tert.-Butanol, 180 Teilen Blausäure und 70 Teilen Austauscherharz mit einer Rührgeschwindigkeit von 500 Umdrehungen pro Minute hergestellt und auf 110°C erhitzt. Das Austauscherharz ist ein sulfoniertes, aus Styrol und Divinylbenzol copolymerisiertes Harz, das vor der Verwendung während 20 Stunden bei 100°C im Vakuum entwässert wurde; es hat Gelstruktur und besitzt eine Korngröße von 10 bis 300 Mikrometern. Nach 2 Stunden bei 110°C wird der Katalysator abgetrennt und das Filtrat destilliert. Als Rückstand erhält man 165 Teile N-tert.-Butylformamid (82% der Theorie) vom Siedepunkt 82 bis 85°C/14 mbar.

## Beispiel 4

In einem Rührautoklaven wird eine Suspension von 112 Teilen Isobuten, 216 Teilen Blausäure, 36 Teilen Wasser und 70 Teilen Austauscherharz mit einer Rührgeschwindigkeit von 500 Umdrehungen pro Minute hergestellt und auf 80°C erhitzt. Das Austauscherharz ist ein sulfoniertes, aus Styrol und Divinylbenzol copolymerisiertes Harz, das vor der Verwendung während 20 Stunden bei 100°C im Vakuum entwässert wurde; es hat Gelstruktur und besitzt eine Korngröße von 10 bis 300 Mikrometern. Nach 2 Stunden Rühren bei 80°C wird der Katalysator abgetrennt und das Filtrat destilliert. Als Rückstand erhält man 185 Teile N-tert.-Butylformamid (92% der Theorie) vom Siedepunkt 82 bis 85°C/14 mbar.

## Beispiel 5

In einem Rührautoklaven wird eine Suspension von 200 Teilen Acetonitril, 120 Teilen tert.-Butanol und 50 Teilen Austauscherharz mit einer Rührgeschwindigkeit von 500 Umdrehungen pro Minute hergestellt und auf 120°C erhitzt. Das Austauscherharz ist ein sulfoniertes, aus Styrol und Divinylbenzol copolymerisiertes Harz, das vor der Verwendung während 20 Stunden bei 100°C im Vakuum entwässert wurde; es hat Gelstruktur und besitzt eine Korngröße von 20 bis 200 Mikrometern. Nach 3 Stunden Rühren bei 120°C werden stündlich 40 Teile Acetonitril und 25 Teile tert.-Butanol bei 120°C zugeführt und entsprechend 65 Teile Suspension über eine Absaugleitung mit einem Metallfilter filtriert und einer Destillation zugeführt. Während 10 Stunden Betrieb erhält man nach der destillativen Abtrennung der nicht umgesetzten Ausgangsstoffe 585 Teile N-tert.-Butylacetamid (88% der Theorie) vom Fp 97 bis 98°C.

**Patentanspruch**

Verfahren zur Herstellung von N-substituierten Carbonsäureamiden der Formel

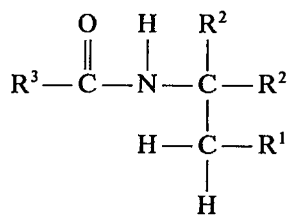

(I)

worin die einzelnen Reste $R^1$ und $R^2$ gleich oder verschieden sein können und jeweils einen aliphatischen Rest bedeuten, zwei der Reste $R^1$ und $R^2$ auch jeweils für ein Wasserstoffatom stehen können, $R^3$ ein Wasserstoffatom, einen aliphatischen, araliphatischen oder aromatischen Rest bezeichnet, durch Umsetzung von Olefinen mit Cyanverbindungen, dadurch gekennzeichnet, daß man Olefine der Formel

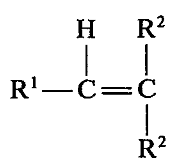

(II)

worin $R^1$ und $R^2$ die vorgenannte Bedeutung besitzen, mit Cyanverbindungen der Formel

$$R^3CN \qquad (III)$$

worin $R^3$ die vorgenannte Bedeutung besitzt, und Wasser in Gegenwart von organischen, sulfonsauren

Kationenaustauschern in einer Menge von 10 bis 80 Gewichtsprozent Austauscher, bezogen auf Ausgangsstoff II, umsetzt.

**Claim**

A process for the preparation of an N-substituted carboxylic acid amide of the formula

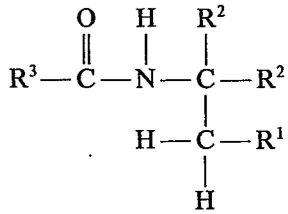

(I)

where the individual radicals $R^1$ and $R^2$ may be identical or different and each is an aliphatic radical, two of the radicals $R^1$ and $R^2$ can also each be hydrogen, and $R^3$ is hydrogen or an aliphatic, araliphatic or aromatic radical, by reaction of an olefin with a cyano compound, characterized in that an olefin of the formula

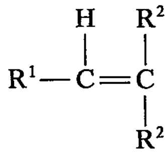

(II)

where $R^1$ and $R^2$ have the above meanings, is reacted with a cyano compound of the formula

$$R^3CN \qquad (III)$$

where $R^3$ has the above meaning, and with water, in the presence of from 10 to 80 per cent by weight, based on starting material II, of an organic cation exchanger containing sulfonic acid groups.

**Revendication**

Procédé de préparation d'amides d'acides carboxyliques N-substitués de la formule

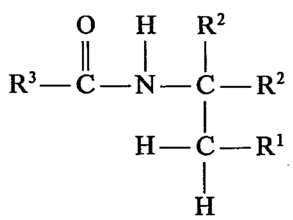

(I)

dans laquelle les différents restes $R^1$ et $R^2$ peuvent être identiques ou différents et désignent chacun un reste aliphatique, deux des restes $R^1$ et $R^2$ pouvant aussi être des atomes d'hydrogène, et $R^3$ désigne un atome d'hydrogène ou un reste aliphatique, araliphatique ou aromatique, par réaction d'oléfines avec des composés cyanés, caractérisé en ce que l'on fait réagir des oléfines de la formule

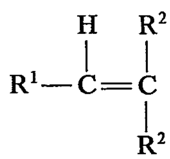

(II)

dans laquelle $R^1$ et $R^2$ possèdent les significations définies, avec des composés cyanés de la formule

$$R^3CN \qquad (III)$$

dans laquelle $R^3$ possède les significations définies, et de l'eau en présence d'échangeurs de cations organiques sulfonés en une proportion de 10 à 80% en poids par rapport au composé de départ II.